# EUROPÄISCHE PATENTSCHRIFT

(11) **EP 1 274 658 B1**
(45) Veröffentlichungstag und Bekanntmachung des Hinweises auf die Patenterteilung: **24.11.2010**
(21) Anmeldenummer: 01935941.3
(22) Anmeldetag: 03.04.2001
(51) Int. Cl.: C03C 8/02, C03C 12/00, A61K 6/06

(54) **SILIKATGLAS ZUR MODIFIKATION KERAMISCHER WERKSTOFFE UND VERWENDUNG EINES SOLCHEN SILIKATGLASES**
SILICATE GLASS FOR MODIFYING CERAMIC MATERIALS AND THE USE OF A SILICATE GLASS OF THIS TYPE
VERRE SILICATE DESTINE A LA MODIFICATION DE MATERIAUX CERAMIQUES ET SON UTILISATION

(30) Priorität: 03.04.2000 DE 10016057
(43) Veröffentlichungstag der Anmeldung: 15.01.2003
(73) Patentinhaber: Merz Dental GmbH, 24321 Lütjenburg (DE)
(72) Erfinder: MÖNKMEYER, Ulrich, 07760 Cala d'Or (ES)
(74) Vertreter: Kunz, Herbert
(86) Internationale Anmeldenummer: PCT/DE2001/001287
(87) Internationale Veröffentlichungsnummer: WO 2001/074730

(56) Entgegenhaltungen:
- WO-A-97/45377
- DE-A- 3 902 771
- DE-A- 19 502 144
- US-A- 6 022 819

## Beschreibung

Die Erfindung betrifft ein Silikatglas zur Modifikation keramischer Werkstoffe, insbesondere von auf natürlichen Feldspatkeramikarten basierenden Werkstoffen, sowie die Verwendung eines solchen Silikatglases zur Beimischung zu keramischen Werkstoffen, insbesondere zu auf natürlichen Feldspatkeramikarten basierenden Werkstoffen.

Keramische Werkstoffe finden immer weitere Anwendungsgebiete. Aufgrund ihrer extremen Härte, aber auch aufgrund ihres Aussehens verdrängen sie teilweise metallische Werkstoffe oder dienen zur Beschichtung metallischer Träger. Ein klassisches Beispiel einer solchen Anwendung sind künstliche Zähne, wie sie unter anderem für Kronen oder Brücken im Dentalbereich Verwendung finden. Darüber hinaus gibt es jedoch eine Vielzahl weiterer Anwendungen, die ständig mehr werden.

Keramische Werkstoffe haben den Nachteil, daß ihre mechanische Festigkeit um den Faktor 100 unter den theoretisch errechneten Werten liegt. Ein Grund dafür sind die in der Griffithschen Bruchmechanik postulierten Mikrorisse. Sie sind mit bis zu 1000 Sprüngen pro cm² in Gläsern und Keramiken vorhanden und wegen ihrer Größe von ca. 3 bis 6 µm ohne optische Hilfsmittel nicht sichtbar.

Insbesondere nach der mechanischen Bearbeitung keramischer Werkstoffe, etwa durch Fräsen, treten vermehrt solche Risse auf. Aber auch bei der Beschichtung anderer Materialien mit Gläsern oder Keramiken tritt Rißbildung auf, da unterschiedliche Wärmeausdehnungen des Trägermaterials und der Keramik die Regel sind. Das Aufbrennen von keramischen Materialien, beispielsweise auf metallene Träger, ist deshalb problematisch. Da bereits Temperaturwechsel mit geringem Temperaturunterschied zu Rißbildung führen können, treten sogenannte Spätsprünge auch nach längerer Zeit auf, und es kommt zu einem Alterungsprozeß des Materials.

Bei herkömmlichen Verfahren wird deshalb versucht, Materialien mit möglichst eng zusammenliegenden Wärmeausdehnungskoeffizienten zu verwenden und durch Temperatur-Zeit-Zyklen beim Brennen die Differenzen auszugleichen.

Sind sichtbare Sprünge vorhanden oder ist die Stabilität des Werkstücks durch Mikrorisse zu stark reduziert, ist dieses unbrauchbar und als Ausschuß zu betrachten. Um brauchbare Ergebnisse zu erhalten, müssen viele verschiedene Parameter gleichzeitig beherrscht werden. Nur Personen mit viel Erfahrung erreichen einen zufriedenstellenden Qualitätsstandard. Zudem ist ein beträchtlicher Arbeits- und Geräteaufwand erforderlich, der die Kalkulation der Hersteller erheblich belastet.

Ein weiteres Problem bei der Oberflächenbeschichtung eines metallischen Trägers mit einem keramischen Werkstoff, wie beispielsweise bei der Herstellung künstlicher Zähne, wie sie für Kronen oder Brücken verwendet werden, liegt in der Verbindung zwischen dem metallischen Grundgerüst und dem kosmetischen Verblendwerkstoff. Herkömmliche Verfahren ermöglichen nur eine inselweise Verbindung zwischen Keramik- und Metallflächen. In die Zwischenräume können daher Flüssigkeiten eindringen, wodurch die Verbindung geschwächt wird und Verfärbungen der Keramik eintreten können.

Aus der WO 97/45377A ist eine Silikatglaszusammensetzung zur Modifikation keramischer Werkstoffe bekannt, mit der die Materialeigenschaften bekannter Keramikmaterialien und insbesondere von Dentalkeramiken modifiziert werden können. Die genannte Silikatglaszusammensetzung soll zu einem Pulver einer durchschnittlichen Teilchengröße zwischen 8 und 12 µm zermahlen und dann weiterverarbeitet werden. Daraus kann jedoch kein Produkt hergestellt werden, das sich mit den herkömmlichen zahntechnischen Vorgehensweisen verarbeiten lässt. Außerdem ist die Anwendung auf wenige Keramikmaterialien beschränkt.

Aufgabe der Erfindung ist es, ein Silikatglas zur Modifikation keramischer Werkstoffe anzugeben, mit dem die Materialeigenschaften bekannter Keramikmaterialien, insbesondere von natürlichen Feldspatkeramikarten, verbessert werden können.

Die Aufgabe wird erfindungsgemäß durch ein Silikatglas zur Modifikation keramischer Werkstoffe gelöst, das dadurch gekennzeichnet ist, dass es durch Zusammenschmelzen hochreiner synthetischer Oxide gewonnen und zu einem Pulver mit einer Korngröße zwischen 20 und 30 µm oder einer Korngröße zwischen 25 und 35 µm zermahlen ist.

Herkömmliche keramische Werkstoffe werden immer aus natürlichem Feldspat gewonnen. Die natürlichen Ausgangsstoffe werden hochgradig gereinigt. Trotzdem verbleiben immer Verunreinigungen. Dadurch entstehen kristalline Bereiche, die durch mechanische Spannungen bei Temperaturänderungen oder mechanischer Bearbeitung zu Mikrorissen führen.

Das erfindungsgemäße Silikatglas verfügt über keinerlei kristalline Bereiche, da es aus hochreinen, synthetisch hergestellten Oxiden gewonnen wird. Verunreinigungen sind deshalb auszuschließen.

Darüber hinaus wird das erfindungsgemäße Silikatglas, das zunächst in Form eines amorphen Glasbrockens vorliegt, zu einem Pulver mit einer Korngröße zwischen 20 und 30 µm oder zwischen 25 und 35 µm zermahlen. Mit diesem Pulver kann dann ein keramischer Werkstoff, insbesondere ein auf natürlichen Feldspatkeramikarten basierender keramischer Werkstoff, erfolgreich modifiziert werden. Durch die gleichmäßige Korngröße zwischen 20 und 30 µm oder zwischen 25 und 35 µm kann eine gleichmäßige Verteilung des erfindungsgemäßen Silikatglases bei der Modifikation keramischer Werkstoffe erreicht werden. In den herkömmlichen keramischen Werkstoffen vorhandene zusammenhängende kristalline Bereiche werden erfolgreich verkleinert und isoliert, so daß die Rißbildung erheblich eingeschränkt wird.

Die zu wählende Krongröße hängt auch vom zu modifizierenden Werkstoff ab. Je nach keramischem Werkstoff eignet sich eine gleichmäßige Korngröße des erfindungsgemäßen Silikatglases zwischen 20 und 30 µm oder 25 - 35 µm besonders gut zur Herbeiführung dieses Effekts. Vorzugsweise werden für das erfindungsgemäße Silikatglas die nachfolgend aufgelisteten synthetischen Oxide mit den weiterhin angegebenen Anteilen, bezogen auf die Gewichtsbasis, verwendet:

| | | | |
|---|---|---|---|
| SiO₂ | 30 | bis | 80% |
| Al₂O₃ | 5 | bis | 20% |
| R₂O | 5 | bis | 35% |
| R'O | 0,5 | bis | 8% |

Dabei steht R für ein oder mehrere Alkalimetalle, vorzugsweise Natrium oder Kalium, und R' für ein oder mehrere Erdalkalimetalle, vorzugsweise Calcium oder Magnesium.

In einer bevorzugten Zusammensetzung enthält das erfindungsgemäße Silikatglas bezogen auf Gewichtsbasis weiterhin einen oder mehrere der folgenden Bestandteile:

| | | |
|---|---|---|
| B₂O₃ | bis | 18% |
| ZrO₂ | bis | 15% |
| SnO₂ | bis | 5% |
| TiO₂ | bis | 10% |

Vorzugsweise beträgt der Anteil wenigstens eines dieser Bestandteile 0,5 Gew.-% oder mehr.

Dem erfindungsgemäßen Silikatglas können feingemahlene Farbpigmente beigemischt sein, um die optische Erscheinung der Oberfläche zu verbessern und die Farbstabilität bei Brennvorgängen zu erhöhen. Dabei werden die Farbpigmente vorzugsweise auf eine Korngröße von unter 20 µm Durchmesser zermahlen.

Das erfindungsgemäße Silikatglas weist einen niedrigen Schmelzpunkt auf. Dies ist insbesondere für die Beschichtung eines Trägers mit einem keramischen Werkstoff wünschenswert, damit die Struktur des Trägermaterials nicht durch übermäßige Wärmeeinflüsse geschädigt wird.

Weiterhin weist das erfindungsgemäße Silikatglas eine hohe Benetzungsfähigkeit auf, die bei der Beschichtung beispielsweise eines metallischen Trägermaterials erforderlich ist. So wird die inselweise Verbindung der Keramikbeschichtung mit dem Trägermaterial verbessert. Eine vollflächige Verbindung wird erleichtert. Die Beschichtung ist haltbarer und auch nach längerer Zeit noch optisch ansprechend.

Weiterhin ist eine hohe Säurefestigkeit des erfindungsgemäßen Silikatglases gegeben.

Je nach Zusammensetzung des Silikatglases kann ein vorgegebener Wärmeausdehnungskoeffizient erreicht werden. Es ist also möglich, durch entsprechende Auswahl der Zusammensetzung des Silikatglases und Modifikation eines herkömmlichen keramischen Werkstoffes mit dem erfindungsgemäßen Silikatglas den Wärmeausdehnungskoeffizienten genauer als bisher zu steuern, so daß die Rißbildung auch auf diesem Wege verhindert werden kann.

Das erfindungsgemäße Silikatglas sorgt bei der Modifikation keramischer Werkstoffe auch dafür, daß bereits entstandene oder später noch entstehende Risse durch Diffusion wieder verschlossen werden. Durch die vollständig amorphe Struktur des erfindungsgemäßen Silikatglases werden Diffusionstiefen von ungefähr 50 µm erreicht. Die nachträgliche Modifikation bereits gebrannter keramischer Werkstoffe ist deshalb ebenfalls möglich.

Eine weitere Aufgabe der Erfindung ist die Angabe einer vorteilhaften Verwendung eines im vorstehenden beschriebenen Silikatglases.

Erfindungsgemäß wird diese Aufgabe durch die Verwendung eines Silikatglases zur Beimischung für keramische Werkstoffe mit einem Anteil von 2 bis 25 Gew.-%.

Das erfindungsgemäße Silikatglas kann vorzugsweise zur Modifikation von auf natürlichen Feldspatarten basierenden Werkstoffen verwendet werden. Die Beimischung kann je nach Zielrichtung der Modifikation zwischen 2 und 12 Gew.-% oder zwischen 12 und 25 Gew.-% betragen.

Bei einer Beimischung im Bereich zwischen 2 und 12 Gew.-% wird eine Stabilisierung des Wärmeausdehnungskoeffizienten erreicht. Bei den herkömmlichen keramischen Werkstoffen ist der Wärmeausdehnungskoeffizient über die in der Praxis benötigten Temperaturbereiche nicht konstant, sondern hängt von der Temperatur ab. Dies führt dazu, daß selbst bei einem sorgfältig auf das metallische Trägermaterial abgestimmten Keramikmaterial beim Aufbrennen des keramischen Werkstoffes innerhalb des durchschrittenen Temperaturbereichs eine Abweichung zwischen Wärmeausdehnungskoeffizient des Keramikmaterials und Wärmeausdehnungskoeffizient des Trägermaterials vorliegt. Dadurch entstehen Mikrocracks im Keramikmaterial.

Durch die Beimischung des erfindungsgemäßen Silikatglases in einem Anteil von 2 bis 12% Gew.-% wird der Wärmeausdehnungskoeffizient über weite Temperaturbereiche stabilisiert, so daß Mikrocracks bereits bei und nach dem Brennvorgang verhindert werden. Dennoch entstehende Mikrocracks werden durch die Diffusionseigenschaften des Modifikationswerkstoffes nachträglich verschlossen.

Darüber hinaus wird durch die Modifikation eines keramischen Werkstoffes mit dem erfindungsgemäßen Silikatglas im Bereich zwischen 2 und 12 Gew.-% die Farbe des keramischen Werkstoffes verbessert und stabilisiert. Der Brennprozeß führt zu keinerlei Veränderung von Chroma (Farbsättigung), Value (Helligkeitswert) und Hue (Grauwert) des Keramikmaterials.

Dies ist insbesondere bei der Herstellung von künstlichen Zähnen von Vorteil, da es wesentlich auf die nach dem Brennvorgang erreichten Farbwerte ankommt.

Auch die Formstabilität eines keramischen Werkstoffes kann durch die Verwendung eines erfindungsgemäßen Silikatglases zur Modifikation mit einem Anteil von 2 bis 12% Gew.-% wesentlich verbessert werden. Beispielsweise bei der Herstellung von künstlichen Zähnen wird auf einem Metallgrundkörper Keramikmaterial in mehreren Schichten aufgebrannt. Dabei wird zunächst eine Schicht aus stark opakem Material aufgebaut und gebrannt. Auf diese das Metall deckende Schicht wird dann eine weitere Schicht aus durchscheinendem Material aufgetragen. Vor dem abschließenden Brennen kann noch eine weitere Schicht aus stärker durchscheinendem Material als Schneidefläche aufgetragen werden, wodurch das natürliche Aussehen der künstlichen Zähne weiter verstärkt wird.

Das Brennen erfolgt jeweils bei Temperaturen zwischen 650° und 1100° C. Mit herkömmlichen Materialien tritt jedoch immer eine mehr oder weniger starke Kantenrundung auf, so daß eine umfangreiche Nachbearbeitung erforderlich ist. Die mechanische Bearbeitung führt jedoch, wie bereits oben dargelegt, zu weiteren Rissen im Keramikmaterial. Die Vermeidung von Kantenrundungen bei mehreren aufeinanderfolgenden Brennprozessen trägt also wesentlich dazu bei, die Materialqualität zu steigern.

In herkömmlichen Keramikmaterialien bilden sich immer Leucitkristalle. Durch die Beimischung des erfindungsgemäßen Silikatglases, das vollständig amorph ist, werden die Leucitkristalle auf relativ kleine Größen reduziert und unterliegen einer gleichmäßigen Größenverteilung. Die Keramik wird unter Druckspannung gehalten. Das modifizierte Material weist eine geringere Empfindlichkeit auf Veränderungen der Abkühldauer auf. Der Brennprozeß muß also weniger stark überwacht werden. Auch bei Abweichungen oder Fehlberechnungen der Abkühlzeit oder fehlerhafter Bedienung von Brennöfen usw. entsteht weniger Ausschuß.

Durch die erhöhte Benetzungsfähigkeit wird eine bessere Haftverbindung zum Metallträger erreicht.

Durch die erhebliche Reduzierung von Mikrocracks wird die Bruchfestigkeit des modifizierten keramischen Werkstoffes erheblich verbessert.

Bei einer Beimischung des erfindungsgemäßen Silikatglases zu einem keramischen Werkstoff in einem Anteil von 12 bis 25 Gew.-% werden die oben erläuterten Eigenschaften bei einer Beimischung von unter 12 Gew.-% nicht wesentlich geändert. Insbesondere bleibt die Säurebeständigkeit erhalten. Zusätzlich wird jedoch die Brenntemperatur um bis zu 200° C gesenkt. Dies wirkt sich besonders vorteilhaft bei dem Aufbrennen eines modifizierten keramischen Werkstoffes auf einen metallischen Träger aus.

Eine weitere Verwendung des erfindungsgemäßen Silikatglases besteht in der nachträglichen Behandlung eines keramischen Werkstückes dadurch, daß das erfindungsgemäße Silikatglas auf das zu behandelnde Werkstück aufgetragen und bei einer Temperatur von unter 880° C gebrannt wird.

Ein bereits fertiges keramisches Werkstück, das in die gewünschte Form gebracht wurde, oder ein mit einem keramischen Werkstoff beschichteter Träger aus einem anderen Material, kann nachträglich mit dem erfindungsgemäßen Silikatglas modifiziert werden. Dafür wird das Silikatglas in geeigneter Weise auf das zu behandelnde Werkstück aufgetragen, wie beispielsweise dadurch, daß es auf übliche Weise in eine pastöse Form gebracht und auf das zu behandelnde Werkstück aufgepinselt oder aufgespritzt wird. Danach wird das Werkstück bei einer Temperatur von unter 880° C gebrannt. Die Brenntemperatur kann niedrig ausfallen, da das erfindungsgemäße Silikatglas einen niedrigen Schmelzpunkt aufweist. Die Oberflächenhaftung wird durch die hohe Benetzungsfähigkeit des erfindungsgemäßen Silikatglases erleichtert. Das Silikatglas verschließt beim Brennvorgang Mikrosprünge und thermische Spannungssprünge sowie durch mechanische Bearbeitung hervorgerufene Sprünge, auch Makrosprünge, und Schrumpfungsrisse. Durch die vollständig amorphe Struktur diffundiert das aufgebrannte Silikatglas auch nach dem Abkühlen in noch bestehende oder später entstehende Mikrosprünge ein und schließt diese.

Bei Verwendung eines Silikatglases, dem Farbpigmente beigemischt sind, erfolgt durch die Diffusion darüber hinaus ein Transport von Farbpigmenten in den zu modifizierenden Werkstoff hinein. Eine nachträgliche Veränderung der Farbgebung eines Werkstoffs wird so möglich. Außerdem wird die Farbtiefe verbessert.

Durch das Verschließen der Makro- und Mikrosprünge wird die Bruchfestigkeit eines Materials erheblich verbessert. Die Ätzfähigkeit des Materials wird dadurch jedoch nicht beeinträchtigt.

Im folgenden werden verschiedene Anwendungen des erfindungsgemäßen Silikatglases anhand einiger Beispiele näher erläutert:

### Anwendungsbeispiel 1:

Durch Beimischung des erfindungsgemäßen Si-Glases nach Rezeptur 1 in eine bekannte, dentale Metallkeramik im Gewichtsverhältnis 1 : 0,2 wird deren Gefüge entscheidend verbessert. Durch die Bildung kleinerer und homogener verteilter Kristalle, sowie durch das Schließen von Rissen und Poren im Mikrogefüge wird die Biegefestigkeit nach ISO 9693 um den Faktor 1,8 bis 3,8 (je nach Ausgangsgefüge) verbessert.

Außerdem wird bei der modifizierten Beispielkeramik die Toleranzbreite für die Abweichung der Entwicklung der Ausdehnungskoeffizienten um < 17 % und 21 % > vergrößert und die Brenntemperatur um 110°C abgesenkt.

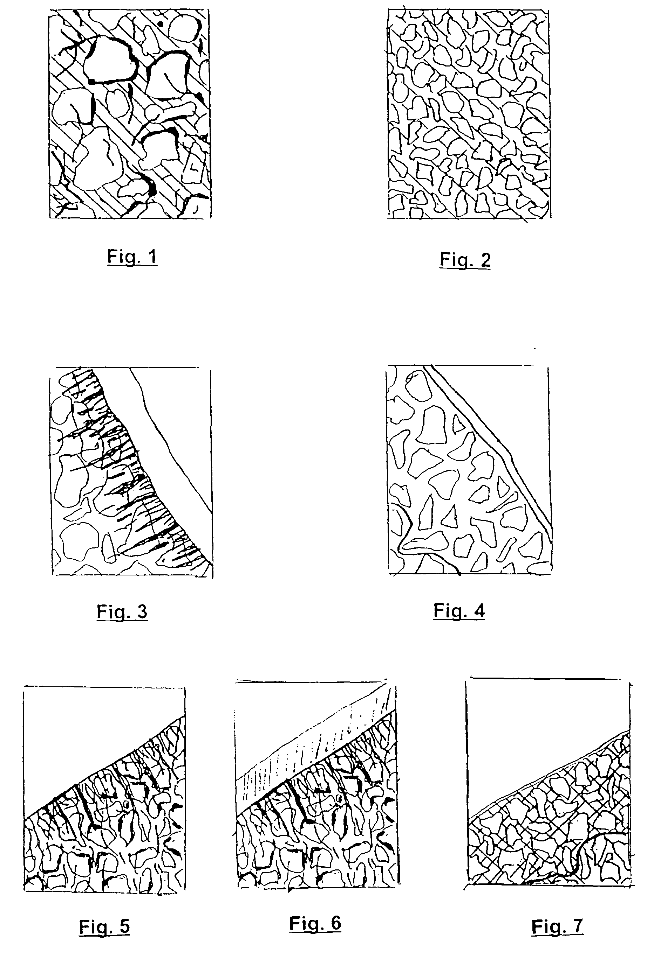

Die Verbesserung des Gefüges und das Verschließen von Rissen ist in den beigefügten **Figuren 1 und 2** dargestellt.

### Anwendungsbeispiel 2:

Durch Auftragen und Brennen des erfindungsgemäßen Glases nach Rezeptur 2, dem erfindungsgemäß Farbpigmente beigemischt werden, werden die durch herkömmliche Keramikmalfarben Microcracks vermieden. Gleichzeitig werden eventuell vorhandene Risse und Poren im Gefüge geschlossen. Außerdem werden die Farbpigmente bis zu 300 µm tief in die Keramikmasse transportiert. Darüber hinaus wird die Biegefestigkeit nach ISO 9693 der Beispielkeramik um den Faktor 1,9 erhöht. Gleichzeitig wird die Keramikmalfarbenschicht an der Oberfläche von durchschnittlich 45 µm auf weniger als 5 µm verkleinert. Die Vermeidung und das Verschließen von Microcracks, Rissen und Poren ist in **Fig. 3** dargestellt. **Fig. 4** zeigt die Keramik-Malfarbenschicht auf der Oberfläche.

### Anwendungsbeispiel 3:

Durch Auftragen und Brennen des erfindungsgemäßen Glases nach Rezeptur 3 werden sowohl die Risse und Poren im Mikrogefüge der Beispielkeramik, als auch die verarbeitungsbedingten Defekte an der Oberfläche geheilt. Dies ist in den **Figuren 5 bis 7** dargestellt. Die Biegefestigkeit der Beispielkeramik nach ISO 9693 wird um den Faktor 2,1 erhöht.

### Anwendungsbeispiel 4:

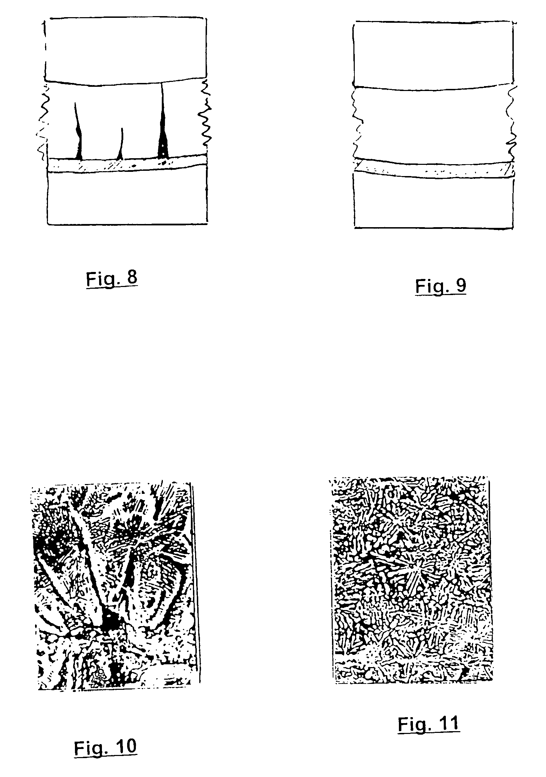

Durch erfindungsgemäße Modifikation einer beispielhaften Aufbrennkeramik (Email) mit einem erfindungsgemäßen Glas nach Rezeptur 4 wird deren Toleranzbreite für die Abweichung in der Entwicklung des Ausdehnungskoeffizienten mit der Temperatur so vergrößert, daß sie auf eine Metallegierung, z. B. Remanium CSE, mit einem um 3,2 x 10⁻⁶ niedrigeren WAK gebrannt werden kann, ohne daß Zugspannungsrisse entstehen. Dies zeigen die **Figuren 8 und 9**. Gleichzeitig wird die Brenntemperatur um 90°C abgesenkt.

### Anwendungsbeispiel 5:

Durch Beimischung des erfindungsgemäßen Si-Glases nach Rezeptur 5 in eine neuartige Dentalkeramik mit hohem Kristallanteil im Gewichtsverhältnis 1:0,05 wird deren Gefüge entscheidend verbessert. Die Kristalle bilden sich kleiner, dichter und gleichmäßiger aus. Wenn durch zusätzliches Auftragen und Brennen des erfindungsgemäßen Si-Glases nach Rezeptur 2 im Anschluß an die Bearbeitung die durch die Bearbeitung entstandenen Cracks verschlossen werden, erhöht sich die Biegefestigkeit nach ISO 9693 bei dieser Beispielkeramik auf ca. 400 MPa **(****Figuren 10 und 11****).**

### Rezepturen

| | **Rezept 1** | **Rezept 2** | **Rezept 3** | **Rezept 4** | **Rezept 5** |
|---|---|---|---|---|---|
| **SiO₂** | 66,7 | 70 | 63 | 66 | 35 |
| **Al₂O₃** | 3 | 8 | 10 | 12 | 12 |
| **K₂O** | 7,7 | 1,7 | 15 | 13 | 20 |
| **Na₂O** | 0,8 | 7 | 6 | 3 | 10 |
| **Li₂O** | 2,6 | 0 | 0 | 2 | 5 |
| **CaO** | 0 | 3,6 | 3 | 0 | 5 |
| **MgO** | 0,6 | 1,7 | 2,5 | 0 | 3 |
| **B₂O₃** | 18,0 | 6,5 | 0,5 | 0 | 0 |
| **ZrO₂** | 0 | 0 | 0 | 0 | 8 |
| **TiO₂** | 0 | 0 | 0 | 3,5 | 2 |
| **SnO₂** | 0,6 | 1,5 | 0 | 0 | 0 |

## Patentansprüche

1. Silikatglas zur Modifikation keramischer Werkstoffe, insbesondere von auf natürlichen Feldspatkeramikarten basierender Werkstoffe, **dadurch gekennzeichnet, dass** das Silikatglas durch Zusammenschmelzen hochreiner synthetischer Oxide gewonnen ist und zu einem Pulver mit einer Korngröße zwischen 20 und 30 µm oder einer Korngröße zwischen 25 und 35 µm zermahlen ist.

2. Silikatglas nach Anspruch 1, **dadurch gekennzeichnet, daß** es bezogen auf Gewichtsbasis folgende Bestandteile enthält:
| | | | |
|---|---|---|---|
| SiO₂ | 30 | bis | 80% |
| Al₂O₃ | 5 | bis | 20% |
| R₂O | 5 | bis | 35% |
| R'O | 0,5 | bis | 8% |
wobei R ein oder mehrere Alkalimetalle, vorzugsweise Natrium oder Kalium, und R' ein oder mehrere Erdalkalimetalle, vorzugsweise Calcium oder Magnesium, sind.

3. Silikatglas nach einem der Ansprüche 1 oder 2, **dadurch gekennzeichnet, daß** es bezogen auf Gewichtsbasis einen oder mehrere der folgenden Bestandteile enthält:
| | | |
|---|---|---|
| B₂O₃ | bis | 18% |
| ZrO₂ | bis | 15% |
| SnO₂ | bis | 5% |
| TiO₂ | bis | 10% |

4. Silikatglas nach Anspruch 3, **dadurch gekennzeichnet, daß** es wenigstens einen der Bestandteile B₂O₃, ZrO₂, SnO₂ oder TiO₂ in einem Anteil bezogen auf Gewichtsbasis von 0,5% enthält.

5. Silikatglas nach einem der Ansprüche 1 bis 4, **dadurch gekennzeichnet, daß** dem Silikatglas Farbpigmente beigemischt sind.

6. Silikatglas nach Anspruch 5, **dadurch gekennzeichnet, daß** die Farbpigmente feingemahlen sind, vorzugsweise mit einer Korngröße unter 20 µm.

7. Verwendung eines Silikatglases nach einem der Ansprüche 1 bis 6 zur Beimischung für keramische Werkstoffe, insbesondere für auf natürlichen Feldspatarten basierenden Werkstoffen, in einem Anteil von 2 bis 25 Gew.-%.

8. Verwendung eines Silikatglases nach einem der Ansprüche 1 bis 7 zur nachträglichen Behandlung eines keramischen Werkstückes, **dadurch gekennzeichnet, daß** das Silikatglas auf das zu behandelnde Werkstück aufgetragen und bei einer Temperatur von unter 880° C, insbesondere im Bereich zwischen 680°C und 850°C, gebrannt wird.

## Claims

1. A silicate glass for modifying ceramic materials, in particular materials based upon natural feldspar ceramic types, **characterised in that** the silicate glass is obtained by fusing together very pure synthetic oxides and is ground to form a powder with a grain size of between 20 and 30 µm or a grain size of between 25 and 35 µm.

2. The silicate glass according to Claim 1, **characterised in that** in relation to the basis weight it contains the following components:
| | |
|---|---|
| SiO₂ | 30 to 80 % |
| Al₂O₃ | 5 to 20 % |
| R₂O | 5 to 35 % |
| R'O | 0.5 to 8 % |
R being one or more alkali metals, preferably sodium or potassium,
and R' being one or more earth alkali metals, preferably calcium or magnesium.

3. The silicate glass according to either of Claims 1 or 2, **characterised in that** in relation to the basis weight it contains one or more of the following components:
| | |
|---|---|
| B₂O₃ | Up to 18 % |
| ZrO₂ | Up to 15 % |
| SnO₂ | Up to 5 % |
| TiO₂ | Up to 10 % |

4. The silicate glass according to Claim 3, **characterised in that** it contains at least one of the components B₂O₃, ZrO₂, SnO₂ or TiO₂ in a proportion in relation to a basis weight of 0.5 %.

5. The silicate glass according to any of Claims 1 to 4, **characterised in that** pigments are incorporated into the silicate glass.

6. The silicate glass according to Claim 5, **characterised in that** the pigments are finely ground, preferably having a grain size of less than 20 µm.

7. The use of a silicate glass according to any of Claims 1 to 6 for the incorporation for ceramic materials, in particular for materials based on natural feldspar types, in a proportion of 2 to 25 % by weight.

8. The use of a silicate glass according to any of Claims 1 to 7 for the subsequent treatment of a ceramic workpiece, **characterised in that** the silicate glass is applied to the workpiece to be treated and fired at a temperature of below 880°C, in particular in the range between 680°C and 850°C.

## Revendications

1. Verre silicaté destiné à la modification de matériaux céramiques, notamment de matériaux à base de céramiques feldspathiques naturelles, **caractérisé en ce que** le verre silicaté est obtenu par la fusion d'oxydes synthétiques de grande pureté et est broyé en une poudre dont les grains ont une grosseur allant de 20 à 30 µm ou une grosseur allant de 25 à 35 µm.

2. Verre silicaté selon la revendication 1, **caractérisé en ce qu'**il contient, sur une base pondérale, les composants suivants :
| | |
|---|---|
| SiO₂ | de 30 à 80 % |
| Al₂O₃ | de 5 à 20 % |
| R₂O | de 5 à 35 % |
| R'O | de 0,5 à 8 % |
dans lesquels R représente un ou plusieurs métaux alcalins, de préférence le sodium ou le potassium, et R' représente un ou plusieurs métaux alcalino-terreux, de préférence le calcium ou le magnésium.

3. Verre silicaté selon l'une des revendications 1 ou 2, **caractérisé en ce qu'**il contient, sur une base pondérale, un ou plusieurs des composants suivants :
| | |
|---|---|
| B₂O₃ | jusqu'à 18 % |
| ZrO₂ | jusqu'à 15 % |
| SnO₂ | jusqu'à 5 % |
| TiO₂ | jusqu'à 10 % |

4. Verre silicaté selon la revendication 3, **caractérisé en ce qu'**il contient au moins un des composants B₂O₃, ZrO₂, SnO₂ ou TiO₂ dans une proportion, sur une base pondérale, de 0,5 %.

5. Verre silicaté selon l'une des revendications 1 à 4, **caractérisé en ce que** des pigments de couleur sont ajoutés au verre silicaté.

6. Verre silicaté selon la revendication 5, **caractérisé en ce que** les pigments de couleur sont finement moulus, en présentant de préférence une grosseur de grains inférieure à 20 µm.

7. Utilisation d'un verre silicaté selon l'une des revendications 1 à 6 en tant qu'élément de mélange dans des matériaux céramiques, notamment des matériaux à base de feldspaths naturels, dans une proportion allant de 2 à 25 % en poids.

8. Utilisation d'un verre silicaté selon l'une des revendications 1 à 7 pour le traitement ultérieur d'une pièce céramique, **caractérisé en ce que** le verre silicaté est appliqué sur la pièce à traiter puis est cuit à une température inférieure à 880 °C, comprise notamment dans la plage de 680 °C à 850 °C.
